# EUROPÄISCHE PATENTSCHRIFT

(11) **EP 1 146 863 B1**
(45) Veröffentlichungstag und Bekanntmachung des Hinweises auf die Patenterteilung: **12.05.2004**
(21) Anmeldenummer: 00906226.6
(22) Anmeldetag: 26.01.2000
(51) Int. Cl.: A61K 9/19, A61K 31/165, A61K 31/155, A61K 31/40

(54) **LYOPHILISATE MIT VERBESSERTER REKONSTITUIERBARKEIT**
LYOPHILISATES WITH IMPROVED RECONSTITUTIBILITY
LYOPHILISATS A RECONSTITUTION AMELIOREE

(30) Priorität: 28.01.1999 DE 19903275
(43) Veröffentlichungstag der Anmeldung: 24.10.2001
(73) Patentinhaber: MERCK PATENT GmbH, 64293 Darmstadt (DE)
(72) Erfinder: KURZ, Thekla, D-64285 Darmstadt (DE); KRÜGER, Ludwig, D-63741 Aschaffenburg (DE); HESSE, Brigitte, D-64407 Fränkisch-Crumbach (DE); KARNATZ, Arnd, D-64380 Rossdorf (DE)
(86) Internationale Anmeldenummer: PCT/EP2000/000569
(87) Internationale Veröffentlichungsnummer: WO 2000/044354

(56) Entgegenhaltungen:
- US-A- 4 002 748
- US-A- 5 679 645
- FRANKS F: "FREEZE-DRYING OF BIOPRODUCTS: PUTTING PRINCIPLES INTO PRACTICE" EUROPEAN JOURNAL OF PHARMACEUTICS AND BIOPHARMACEUTICS,NL,ELSEVIER SCIENCE PUBLISHERS B.V., AMSTERDAM, Bd. 45, Nr. 3, 1. Mai 1998 (1998-05-01), Seiten 221-229, XP000751942 ISSN: 0939-6411

## Beschreibung

Die vorliegende Erfindung betrifft Lyophilisate mit verbesserter Lösungsgeschwindigkeit und Rekonstituierbarkeit sowie ein Verfahren zu deren Herstellung.

Die Lyophilisation, das Gefriertrocknen, ist ein schon lang bekanntes und oft angewandtes Verfahren zur schonenden Haltbarmachung bestimmter Substanzen, wie zum Beispiel temperaturempfindlicher Lebensmittel oder vor allem Medikamente. Hierbei werden die Substanzen in gefrorenem Zustand getrocknet und lassen sich bei Zugabe von Wasser oder eines anderen Lösungsmittels besonders leicht wieder in den ursprünglichen Zustand versetzen. Bei diesem Verfahren werden im allgemeinen zunächst die Ausgangsprodukte auf Temperaturen bis zu -70° C tiefgefroren. Anschließend werden ihnen während des Trocknungsprozesses, der in druckfesten Behältern (Lyophilisatoren) unter Hochvakuum stattfindet, das Wasser durch Sublimation entzogen, und man erhält die gefriergetrocknete Substanz.

Insbesondere wird die Lyophilisation für die Konservierung von empfindlichen Medikamenten eingesetzt. Denn vor allem bei Medikamenten ist es sehr wichtig, dass sie sich während der Lagerung nicht verändern, d. h. dass sich ihre Struktur nicht verändert, umlagert oder sogar zersetzt, was eine starke Beeinträchtigung im Hinblick auf ihre Wirksamkeit bedeuten würde.

Die US5679645 sowie die US4002748 beschreiben Verfahren zur Herstellung von amorphen Lyopohilisaten mit gegenüber der kristallinen Zubereitung verbesserter Löslichkeit, wobei die wäßrige Lösung der zu lyophilisierenden Substanz in 0,01 - 900 s auf unter -20°C bzw. bei -50 bis -55°C rasch auf unter -48°C abgekühlt wird.

Beim Gefriertrocknen besteht stets das Bemühen, eine möglichst große Menge an Wirkstoff in ein möglichst kleines Volumen einzubringen. Das führt dazu, dass oft Konzentrationen in der Nähe der Sättigungskonzentration des Wirkstoffes eingesetzt werden. Dies ist für die Wirtschaftlichkeit der Prozesse notwendig.

In diesen Fällen kann jedoch oft nach durchgeführter Gefriertrocknung das Lyophilisat nicht partikelfrei rekonstituiert werden, so dass eine parenterale Applikation nicht mehr möglich ist. Dies ist zurückzuführen auf Kristalle, die sich nach Überschreiten der Sättigungslöslichkeit durch das Abkühlen gebildet haben. Die Lösungsgeschwindigkeit von Kristallen ist deutlich langsamer als die amorph vorliegender Moleküle.

Aufgabe der vorliegenden Erfindung war es daher, ein Verfahren zur Herstellung von Lyophilisaten, welche eine verbesserte Lösungsgeschwindigkeit aufweisen und sich partikelfrei rekonstituieren lassen, auch wenn sie nahe der Sättigungskonzentration dosiert werden, bereitzustellen.

Überraschend wurde gefunden, dass eine Erwämung der bereits zum Gefriertrocknungsprozess fertig vorbereiteten Lösungen direkt am Gefriertrockner und eine rasche Abkühlung von dieser erhöhten Temperatur auf die Gefriertemperatur, Lyophilisate erzeugt, die die gewünschten vorteilhaften Eigenschaften erzielen.

Gegenstand der Erfindung ist daher ein Verfahren zur Herstellung von Lyophilisaten mit verbesserter Lösungsgeschwindigkeit, dadurch gekennzeichnet, dass man die bereits zur Lyophilisation abgefüllten entsprechenden Lösungen, welche vorher gegebenenfalls zur Beschleunigung der Lösung der Substanz erwärmt, filtriert - gegebenenfalls sterilfiltriert - und abgefüllt wurden, in den Gefäßen direkt am Gefriertrockner nochmals auf 30° bis 95°C erwärmt und dann die Einfrierphase rasch von dieser erhöhten Temperatur auf die gewünschte tiefe Gefriertrocknungstemperatur durchführt.

Das Merkmal "rasch" bedeutet in diesem Zusammenhang einen Zeitraum von 10 Minuten bis 4 Stunden, vorzugsweise 30 Minuten bis 2 Stunden, ganz besonders bevorzugt 30 Minuten bis 1 Stunde.
Die gewünschte Gefriertrocknungstemperatur kann dabei bis zu -70° C betragen, vorzugsweise wird eine Temperatur von ca. -50 °C angewandt.

Bei dem herkömmlichen Gefriertrocknungsprozess wird die Substanz oder der Wirkstoff zur Beschleunigung der Lösung erwärmt. Nach dem Lösen folgen bei einer Sterilherstellung, welche bei Medikamenten üblich ist, die Schritte der Sterilfiltration und der Abfüllung. Diese beiden Schritte können, je nach Größe des Ansatzes, einige Stunden dauern. Die Lösungen kühlen dabei automatisch auf Raumtemperatur ab. Die Beschickung des Gefiertrockners geschieht dann somit bei Raumtemperatur und die Einfrierphase erfolgt darauf so schnell wie möglich von Raumtemperatur auf ca. -50° C. Anschließend beginnt die Trockenphase am Gefriertrockner.

Bei dem erfindungsgemäßen Prozess nun erfolgen die Lösung, Filtration bzw. Sterilfiltration sowie die Abfüllung analog des bekannten Prozesses. Dann wird jedoch bei Raumtemperatur der Gefriertrockner mit den entsprechend vorbereiteten Gefäßen beschickt, und diese Gefäße (vials) im Gerät nochmals erwärmt auf 30° - 95° C. Von dieser erhöhten Temperatur aus wird die Einfrierphase gestartet und so schnell wie möglich auf die gewünschte Gefriertemperatur gebracht. Die Trockenphase erfolgt dann wie üblich.

Durch das erneute Erwärmen der Lösungen wird die Sättigungslöslichkeit deutlich erhöht, was auf die Verkleinerung der Wasercluster zurückzuführen ist. Die erhöhte Löslichkeit führt somit zu einer verbesserten Hydratisierung. Beim raschen Abkühlen fehlt einerseits für die Wassermoleküle die Zeit, größere Cluster zu bilden, andererseits fehlt für die Wirkstoffmoleküle die Zeit, sich zu Kristallkeimen anzuordnen. Das erhaltene Produkt ist dementsprechend amorph und lässt sich partikelfrei rekonstituieren.

Die Erwärmung der Lösungen erfolgt auf Temperaturen von 30° bis 95° C, vorzugsweise werden Temperaturen im Bereich von 30° bis 70° C, besonders bevorzugt im Bereich von 30° bis 60°C ausgewählt.

Durch den erfindungsgemäßen Prozess können also deutlich höhere Konzentrationen in ein Volumen eingebracht werden. Dadurch wird die Trocknungszeit vermindert und die Wirtschaftlichkeit der Prozesse erhöht.

Die so hergestellten Lyophilisate zeigen eine verbesserte Lösungsgeschwindigkeit, sie lassen sich partikelfrei rekonstituieren, obwohl sie nahe der Sättigungskonzentration dosiert werden können.

Gegenstand der Erfindung ist auch die Herstellung von Lyophilisaten der Substanz 2-Methyl-5-methylsulfonyl-4-(1-pyrrolyl)-benzoylguanidin-methansulfonat nach dem hier beschriebenen Verfahren (siehe Beispiel 1).
Diese Substanz (EMD 96785), die z.B aus der DE 4430861 bekannt ist, ist ein NHE-Inhibitor, welcher die Na⁺/H⁺-Ionenpumpe in den Myocardzellen blockiert. Damit wird eine Übersäuerung der Zellen beim Infarkt verhindert, die zum Absterben von Myocardgewebe führt.

Gegenstand der Erfindung ist auch die Herstellung von Lyophilisaten der Substanz N-[2-Methyl-4,5-bis(methylsulfonyl)-benzoylguanidinhydrochlorid nach dem hier beschriebenen Verfahren (siehe Beispiel 2).

Diese Substanz (EMD 87580), die z.B aus der EP 0 758 644 A1 bekannt ist, ist ebenfalls ein NHE-Inhibitor, welcher die Na⁺/H⁺lonenpumpe in den Myocardzellen blockiert. Damit wird eine Übersäuerung der Zellen beim Infarkt verhindert, die zum Absterben von Myocardgewebe führt.

Gegenstand der Erfindung ist auch die Herstellung von Lyophilisaten der Substanz 4-Isopropyl-3-methylsulfonyl-benzoylguanidinmethansulfonat nach dem hier beschriebenen Verfahren.

Diese Substanz (Cariporide), die z.B aus der EP 589 336 bekannt ist, ist ebenfalls ein NHE-Inhibitor.

Gegenstand der Erfindung sind ferner pharmazeutische Zubereitungen, enthaltend mindestens ein erfindungsgemäßes Lyophilisat.
Die pharmazeutischen Zubereitungen können als Arzneimittel in der Human- oder Veterinärmedizin verwendet werden. Als Trägerstoffe - kommen organische oder anorganische Substanzen in Frage, die sich für die enterale (z.B. orale), parenterale oder topische Applikation eignen und mit den Lyophilisaten nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, femer Suspensionen, Emulsionen oder Implantate, für die topische Anwendung Salben, Cremes oder Puder. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Vorzugsweise enthalten die Zubereitungen die Lyophilisate z.B. zur Herstellung von Injektionspräparaten.

Auch ohne weitere Ausführungen wird davon ausgegangen, dass ein Fachmann die obige Beschreibung in weitestem Umfang nutzen kann. Die bevorzugten Ausführungsformen sind deswegen lediglich als beschreibende, keineswegs als in irgendeiner Weise limitierende Offenbarung aufzufassen.

### Beispiel 1

a)
   Man löst 100 mg 2-Methyl-5-methylsulfonyl-4-(1-pyrrolyl)-benzoylguanidin-methansulfonat (ein NHE-Inhibitor) in 20 ml Wasser durch Erwärmen auf 40°C, um die Lösung zu beschleunigen. Anschließend wird die Lösung sterilfiltriert und in die für die Gefriertrocknung geeigneten Gefäße (vials) abgefüllt. Dabei kühlt die Lösung auf Raumtemperatur ab.
   Der Gefriertrockner wird bei Raumtemperatur mit den vials beschickt, anschließend werden diese Gefäße nochmals erwärmt auf ca. 50°C. Dann wird innerhalb 1 Stunde von +50°C auf -50°C eingefroren. Die Trocknungsphase verläuft dann wie üblich.
   Die so erhaltenen Lyophilisate sind amorph und lassen sich partikelfrei rekonstituieren.
b) Vergleichsbeispiel
   Wieder werden 100 mg 2-Methyl-5-methylsulfonyl-4-(1-pyrrolyl)-benzoylguanidin-methansulfonat in 20 ml Wasser unter Erwärmen auf 40°C gelöst. Die Lösung wird sterilfiltriert und abgefüllt, wobei die Lösung auf Raumtemperatur abkühlt.
   Dann werden die Gefäße am Gefriertrockner von Raumtemperatur innerhalb 1 Stunde auf -50°C gekühlt und eingefroren.

Bei diesem herkömmlichen Verfahren bekommt man schon während der Lyphilisation eine Kristallbildung, was dazu führt, dass sich die Lyophilisate bei der Rekonstitution nicht vollständig auflösen. Hier musste, um ein zu a) vergleichbares Lyophilisat zu erhalten, die Konzentration auf 50 mg / 20 ml reduziert werden.

Das aber bedeutet, dass bei dem erfindungsgemäßen Verfahren eine höhere Konzentration des Wirkstoffes gewählt werden kann und man dennoch Lyophilisate mit verbesserter Lösungsgeschwindigkeit erhält.

### Beispiel 2:

Man löst 100 mg N-[2-Methyl-4,5-bis(methylsulfonyl)-benzoyl-guanidinhydrochlorid in 10 ml Wasser durch Erwärmen auf 40°C. Anschließend wird die Lösung sterilfiltriert und in die für die Gefriertrocknung geeigneten Gefäße (vials oder Ampullen) abgefüllt. Dabei kühlt die Lösung auf Raumtemperatur ab.

Der Gefriertrockner wird auf -59°C heruntergehkühlt. Die mit der Lösung gefüllten vials werden in einem Trockenschrank auf +40°C erwärmt und anschließend in den schon auf -50°C heruntergekühlten Gefrieretrockner verbracht. Die Lösung wird dabei schnellstmöglich eingefroren. Die Trocknungsphase verläuft dann wie üblich.
Ebenso ist ein Erwärmen der vials im Gefriertrockner und anschließendes Abkühlen ( wie in Beispiel 1 beschrieben) möglich.

## Patentansprüche

1. Verfahren zur Herstellung von Lyophilisaten mit verbesserter Lösungsgeschwindigkeit, **dadurch gekennzeichnet, dass** man die bereits zur Lyophilisation abgefüllten entsprechenden Lösungen, welche vorher gegebenenfalls zur Beschleunigung der Lösung der Substanz erwärmt, filtriert - gegebenenfalls sterilfiltriert - und abgefüllt wurden, in den Gefäßen direkt am Gefriertrockner nochmals auf 30° bis 95°C erwärmt und dann die Einfrierphase rasch von dieser erhöhten Temperatur auf die gewünschte tiefe Gefriertrocknungstemperatur durchführt.

2. Verfahren nach Anspruch 1, **dadurch gekennzeichnet, dass** die Gefäße mit den Lösungen am Gefriertrockner auf 30° bis 60° C erwärmt werden.

3. Verfahren nach Anspruch 1 oder 2, **dadurch gekennzeichnet, dass** Lyophilisate erhalten werden, die sich partikelfrei rekonstituieren lassen.

4. Verfahren nach einem der Ansprüche 1 bis 3, **dadurch gekennzeichnet, dass** Lyophilisate der Substanz 2-Methyl-5-methylsulfonyl-4-(1-pyrrolyl)-benzoylguanidin-methansulfonat, N-[2-Methyl-4,5-bis(methylsulfonyl)-benzoyl]-guanidin-hydrochlorid oder 4-Isopropyl-3-methylsulfonyl-benzoylguanidin-methansulfonat hergestellt werden.

5. Lyophilisate mit verbesserter Lösungsgeschwindigkeit, gekennzeichnet und erhaltlich dadurch, dass man bei der Herstellung der Lyophilisate die bereits zur Lyophilisation abgefüllten entsprechenden Lösungen, welche vorher gegebenenfalls zur Beschleunigung der Lösung der Substanz erwärmt, filtriert oder sterilfiltriert und abgefüllt wurden, in den Gefäßen am Gefriertrockner direkt nochmals auf 30° bis 95°C erwärmt und dann die Einfrierphase rasch von dieser erhöhten Temperatur auf die gewünschte tiefe Gefriertrocknungstemperatur durchführt.

6. Lyophilisate der Substanz 2-Methyl-5-methylsulfonyl-4-(1-pyrrolyl)-benzoylguanidin-methansulfonat mit verbesserter Rekonstituierbarkeit, gekennzeichnet und erhältlich dadurch, dass man bei der Herstellung der Lyophilisate die bereits zur Lyophilisation abgefüllten entsprechenden Lösungen, welche vorher gegebenenfalls zur Beschleunigung der Lösung der Substanz erwärmt, filtriert oder sterilfiltriert und abgefüllt wurden, in den Gefäßen am Gefriertrockner direkt nochmals auf 30° bis 95°C erwärmt und dann die Einfrierphase rasch von dieser erhöhten Temperatur auf die gewünschte tiefe Gefriertrocknungstemperatur durchführt.

7. Lyophilisate der Substanz N-[2-Methyl-4,5-bis(methylsulfonyl)-benzoylguanidin-hydrochlorid mit verbesserter Rekonstituierbarkeit, gekennzeichnet und erhältlich dadurch, dass man bei der Herstellung der Lyophilisate die bereits zur Lyophilisation abgefüllten entsprechenden Lösungen, welche vorher gegebenenfalls zur Beschleunigung der Lösung der Substanz erwärmt, filtriert oder sterilfiltriert und abgefüllt wurden, in den Gefäßen am Gefriertrockner direkt nochmals auf 30° bis 95°C erwärmt und dann die Einfrierphase rasch von dieser erhöhten Temperatur auf die gewünschte tiefe Gefriertrocknungstemperatur durchführt.

8. Lyophilisate der Substanz 4-Isopropyl-3-methylsulfonylbenzoylguanidin-methansulfonat mit verbesserter Rekonstituierbarkeit, gekennzeichnet und erhältlich dadurch, dass man bei der Herstellung der Lyophilisate die bereits zur Lyophilisation abgefüllten entsprechenden Lösungen, welche vorher gegebenenfalls zur Beschleunigung der Lösung der Substanz erwärmt, filtriert oder sterilfiltriert und abgefüllt wurden, in den Gefäßen am Gefriertrockner direkt nochmals auf 30° bis 95°C erwärmt und dann die Einfrierphase rasch von dieser erhöhten Temperatur auf die gewünschte tiefe Gefriertrocknungstemperatur durchführt.

9. Pharmazeutische Zubereitung enthaltend mindestens ein Lyophilisat nach den Ansprüchen 5-8.

## Claims

1. Process for the preparation of lyophilisates having an improved dissolution rate, **characterised in that** the corresponding solutions already drawn off for lyophilisation which have, if necessary, previously been warmed in order to accelerate dissolution of the substance, filtered - optionally sterile-filtered - and drawn off, are re-warmed to 30° to 90°C in the vials directly in the freeze drier, and the freezing phase is then carried out rapidly from this elevated temperature to the desired low freeze-drying temperature.

2. Process according to Claim 1, **characterised in that** the vials containing the solutions are warmed to 30° to 60°C in the freeze drier.

3. Process according to Claim 1 or 2, **characterised in that** lyophilisates which can be reconstituted in a particle-free manner are obtained.

4. Process according to one of Claims 1 to 3, **characterised in that** lyophilisates of the substance 2-methyl-5-methylsulfonyl-4-(1-pyrrolyl)benzoylguanidine methanesulfonate, N-[2-methyl-4,5-bis(methylsulfonyl)benzoyl]guanidine hydrochloride or 4-isopropyl-3-methylsulfonylbenzoylguanidine methanesulfonate are prepared.

5. Lyophilisates having an improved dissolution rate, characterised and obtainable in that, in the preparation of the lyophilisates, the corresponding solutions already drawn off for lyophilisation, which have, if necessary, previously been warmed in order to accelerate dissolution of the substance, filtered or sterile-filtered and drawn off, are directly re-warmed to 30° to 95°C in the vials in the freeze drier, and the freezing phase is then carried out rapidly from this elevated temperature to the desired low freeze-drying temperature.

6. Lyophilisates of the substance 2-methyl-5-methylsulfonyl-4-(1-pyrrolyl)benzoylguanidine methanesulfonate having improved reconstitutability, characterised and obtainable in that, in the preparation of the lyophilisates, the corresponding solutions already drawn off for lyophilisation, which have, if necessary, previously been warmed in order to accelerate dissolution of the substance, filtered or sterile-filtered and drawn off, are directly re-warmed to 30° to 95°C in the vials in the freeze drier, and the freezing phase is then carried out rapidly from this elevated temperature to the desired low freeze-drying temperature.

7. Lyophilisates of the substance N-[2-methyl-4,5-bis(methylsulfonyl)benzoyl]guanidine hydrochloride having improved reconstitutability, characterised and obtainable in that, in the preparation of the lyophilisates, the corresponding solutions already drawn off for lyophilisation, which have, if necessary, previously been warmed in order to accelerate dissolution of the substance, filtered or sterile-filtered and drawn off, are directly re-warmed to 30° to 95°C in the vials in the freeze drier, and the freezing phase is then carried out rapidly from this elevated temperature to the desired low freeze-drying temperature.

8. Lyophilisates of the substance 4-isopropyl-3-methylsulfonylbenzoylguanidine methanesulfonate having improved reconstitutability, characterised and obtainable in that, in the preparation of the lyophilisates, the corresponding solutions already drawn off for lyophilisation, which have, if necessary, previously been warmed in order to accelerate dissolution of the substance, filtered or sterile-filtered and drawn off, are directly re-warmed to 30° to 95°C in the vials in the freeze drier, and the freezing phase is then carried out rapidly from this elevated temperature to the desired low freeze-drying temperature.

9. Pharmaceutical preparation comprising at least one lyophilisate according to Claims 5-8.

## Revendications

1. Procédé pour la préparation de lyophilisats présentant une vitesse de dissolution améliorée, **caractérisé en ce que** les solutions correspondantes déjà soutirées pour la lyophilisation qui ont été, si nécessaire, précédemment chauffées afin d'accélérer la dissolution de la substance, filtrées - optionnellement filtrées de façon stérile - et soutirées, sont re-chauffées jusqu'à 30°C à 90°C dans les fioles directement dans le lyophilisateur, et la phase de congélation est alors mise en oeuvre rapidement à partir de cette température élevée jusqu'à la température de lyophilisation basse souhaitée.

2. Procédé selon la revendication 1, **caractérisé en ce que** les fioles contenant les solutions sont chauffées jusqu'à 30°C à 90°C dans le lyophilisateur.

3. Procédé selon la revendication 1 ou 2, **caractérisé en ce que** des lyophilisats qui peuvent être reconstitués d'une manière exempts de particules sont obtenus.

4. Procédé selon l'une quelconque des revendications 1 à 3, **caractérisé en ce que** des lyophilisats de la substance méthanesulfonate de 2-méthyl-5-méthylsulfonyl-4-(1-pyrrolyl)benzoylguanidine, hydrochlorate de N-[2-méthyl-4,5-bis(méthylsulfonyl)benzoyl]guanidine ou méthanesulfonate de 4-isopropyl-3-méthylsulfonylbenzoylguanidine sont préparés.

5. Lyophilisats présentant une vitesse de dissolution améliorée, caractérisés et pouvant être obtenus en ce que, dans la préparation des lyophilisats, les solutions correspondantes déjà soutirées pour la lyophilisation, qui ont été, si nécessaire, précédemment chauffées afin d'accélérer la dissolution de la substance, filtrées ou filtrées de façon stérile et soutirées, sont re-chauffées jusqu'à 30°C à 95°C dans les fioles dans le lyophilisateur, et la phase de congélation est alors mise en oeuvre rapidement à partir de cette température élevée jusqu'à la température de lyophilisation basse souhaitée.

6. Lyophilisats de la substance méthanesulfonate de 2-méthyl-5-méthylsulfonyl-4-(1-pyrrolyl)benzoylguanidine présentant une capacité de reconstitution améliorée, caractérisés et pouvant être obtenus en ce que, dans la préparation des lyophilisats, les solutions correspondantes déjà soutirées pour la lyophilisation, qui ont été, si nécessaire, précédemment chauffées afin d'accélérer la dissolution de la substance, filtrées ou filtrées de façon stérile et soutirées, sont re-chauffées jusqu'à 30°C à 95°C dans les fioles dans le lyophilisateur, et la phase de congélation est alors mise en oeuvre rapidement à partir de cette température élevée jusqu'à la température de lyophilisation basse souhaitée.

7. Lyophilisats de la substance hydrochlorate de N-[2-méthyl-4,5-bis-(méthylsulfonyl)benzoyl]guanidine présentant une capacité de reconstitution améliorée, caractérisés et pouvant être obtenus en ce que, dans la préparation des lyophilisats, les solutions correspondantes déjà soutirées pour la lyophilisation, qui ont été, si nécessaire, précédemment chauffées afin d'accélérer la dissolution de la substance, filtrées ou filtrées de façon stérile et soutirées, sont re-chauffées jusqu'à 30°C à 95°C dans les fioles dans le lyophilisateur, et la phase de congélation est alors mise en oeuvre rapidement à partir de cette température élevée jusqu'à la température de lyophilisation basse souhaitée.

8. Lyophilisats de la substance méthanesulfonate de 4-isopropyl-3-méthylsulfonylbenzoylguanidine présentant une capacité de reconstitution améliorée, caractérisés et pouvant être obtenus en ce que, dans la préparation des lyophilisats, les solutions correspondantes déjà soutirées pour la lyophilisation, qui ont été, si nécessaire, précédemment chauffées afin d'accélérer la dissolution de la substance, filtrées ou filtrées de façon stérile et soutirées, sont re-chauffées jusqu'à 30°C à 95°C dans les fioles dans le lyophilisateur, et la phase de congélation est alors mise en oeuvre rapidement à partir de cette température élevée jusqu'à la température de lyophilisation basse souhaitée.

9. Préparation pharmaceutique comprenant au moins un lyophilisat selon les revendications 5-8.
